# NEW EUROPEAN PATENT SPECIFICATION

(11) **EP 0 605 015 B2**
(45) Date of publication and mention of the opposition decision: **15.05.2002**
(45) Mention of the grant of the patent: 17.03.1999
(21) Application number: 93121147.8
(22) Date of filing: 30.12.1993
(51) Int. Cl.: D04H 1/00, D04H 1/70, A61F 13/15

(54) **Method and system for making three-dimensional fabrics**
Verfahren und System zur Herstellung von dreidimensionalen Stoffen
Procédé et système de formation de tissus tridimensionels

(30) Priority: 31.12.1992 US 999328
(43) Date of publication of application: 06.07.1994
(73) Proprietor: McNEIL-PPC, INC., Milltown New Jersey 08850 (US)
(72) Inventor: Ulman, John Thomas, Woodbridge, NJ 07095 (US)
(74) Representative: Groening, Hans Wilhelm, Dipl.-Ing.

(56) References cited:
- EP-A- 0 478 182
- EP-A- 0 479 442
- WO-A-92/07985
- FR-A- 2 259 930
- US-A- 5 064 484

## Description

### Field of the Invention

This invention relates to a method and an apparatus for fabricating three-dimensional fabrics for use in sanitary napkins, incontinence pads, surgical dressings or the like. More specifically, the invention relates to forming three-dimensional surfaces such as flexible projections, ridges, wells or the like which may be used to direct fluid within a product, or to provide improved anatomical fit.

### Description of the Prior Art

Products such as sanitary napkins, incontinence pads and surgical dressings often include a layer of absorbent material, and a backing layer or moisture barrier which is impervious to fluid. The absorbent material includes a surface for contacting the body of the consumer or patient, so that body fluids are absorbed into the product and are contained by the moisture barrier. In the case of sanitary napkins and incontinence pads, an adhesive strip may be provided on an outer surface of the moisture barrier for temporarily securing the product to an undergarment of the consumer.

Conventionally, the body contacting surface of the absorbent material in such a product is substantially flat and uniform. Attempts have been made to texture the body-contacting surface by embossing, but embossing tends to compress the absorbent material and lesson its moisture absorbing characteristics. While the potential advantages of a textured body contacting surface, such as greater absorbent surface area and improved anatomical fit are generally recognized, the disadvantages which are inherent in compression type forming techniques such as embossing have, precluded any successful introduction of a textured absorbent product into the marketplace.

EP-A-0 479 442 discloses a method and apparatus for manufacturing absorbent articles having a particulate wad which comprises bonding the wad particles together. Said known method includes providing a discrete wad of finely divided material on a carrier, stabilizing a wad by bonding at least some of the material thereof together, and bonding at least some of the material in the wad to the carrier. Said known apparatus includes a stabilizing and bonding means for stabilizing the wad by bonding at least some of the material thereof together and to the carrier. The carrier may be fluid permeable and the wad may then be formed thereon by a suitable forming means. The carrier is sheet-like and a plurality of discrete wads are formed thereof. These wads are stabilized and bonded to the carrier and the carrier is parted to separate each wad and its associated portion of the carrier from the others. The carrier is parted a predetermined distance from the edge of each wad to provide a border around each wad. The apparatus may include a carrier supply means for supplying the carrier from a roll, and the wads may be formed thereon and bonded thereto in a continuous manner. The wad may be formed by generating a pressure differential across the carrier, defining an area on the carrier through which a transport fluid may pass by means of a former and introducing the finely divided material into the fluid such that the finely divided material is deposited on the carrier in the area defined by the former. The finely divided material is introduced on the high pressure side, and the former being located on the high pressure side of the carrier. The forming means may include a pressure differential generating means for generating a pressure differential across the carrier; a former for defining the desired area on the carrier through which a transporting fluid may pass: and introducing means located on a side of the carrier which is, in use, at a higher pressure, for introducing the finely divided material, in use into a stream of the transporting fluid to be deposited on the carrier in the defined area. The carrier may be supported by a fluid permeable support member when the wad is formed thereon and when the wad is stabilized and bonded to the carrier by applying a binder thereto, which may be a heat curable foamed binder. The binder may be applied on top of the wads and may be drawn into the wads and into the carrier by applying suction from beneath the carrier. The wads and the carrier are then heated to dry and chemically cross-link the binder. Instead, the finely divideed material may include thermoplastic particles and the wad may then be stabilized and bonded to the carrier merely by heating the wad and the carrier so that only a heating means will be necessary. The finely divided material may be fibrous. A cellulosic material such as wood pulp, cotton, rayon or the like may be used. Raw pulp is supplied to a mill from a feed and passes as finely-divided pulp a long supply pipes together with air as the conveying fluid to enter a wad forming station in the shape of a forming box. The pipes enter from opposing sides into the interior of a ring. The ring contains a plurality of apertures passing through the circumference of the ring. The apertures are circular for forming breast pads. A vacuum is supplied to enable a pressure difference to arise between the inside of the ring and the outside of the ring which is covered by a continuous air-permeable liquid-pervious strip of nonwoven fabric. This continuous strip forming the carrier passes around the outside of the ring on a continuous fluid permeable supporting belt. Vacuum is supplied below this supporting belt at a vacuum chamber to maintain the formed wads in position on the air permeable strip. In the wad forming station, the non-woven strip closes off one side of the apertures wereby the finely divided material moves into the apertures and forms a predetermined shape of wad on the strip which is now the carrier. A brush being positioned within or outside the ring brushes off loose fibers which project above the required size of the wad to be formed. Two fiber streams of different compositions can be employed to blend said fibers. Thus, superabsorbent fibers may be mixed in the wad with normal long fiber wood pulp or short fiber wood pulps may be mixed with staple length thermoplastic fibers.

It is clear that there has existed a long and unfilled need in the prior art for a method and apparatus for making a three-dimensional, textured absorbent fabric which does not have diminished moisture absorbing characteristics with respect to conventional, non-textured absorbent products.

### SUMMARY OF THE INVENTION

Accordingly, it is an object of the invention to provide an improved method for manufacturing an absorbent, three-dimensional fabric which exhibits improved moisture absorbency characteristics with respect to conventional non-textured absorbent products.

It is further an object of the invention to provide an apparatus for making a three-dimensional, absorbent fabric which exhibits such improved moisture absorbing characteristics.

In order to achieve the above and other objects of the invention, a method and apparatus of manufacturing a three-dimensional fabric of the type which may be used in absorbent garments, dressings or the like includes the features of claims 1 and 11. US-A-5 064 484 discloses the preamble part of claim 1.

These and various other advantages and features of novelty which characterize the invention are pointed out with particularity in the claims annexed hereto and forming a part hereof. However, for a better understanding of the invention, its advantages, and the objects obtained by its use, reference should be made to the drawings which form a further part hereof, and to the accompanying descriptive matter, in which there is illustrated and described a preferred embodiment of the invention.

### BRIEF DESCRIPTION OF THE DRAWINGS

FIGURE 1 is a diagrammatical view of an apparatus for manufacturing a three-dimensional fabric according to a preferred embodiment of the invention;
FIGURE 2 is a fragmentary perspective view of a contoured screen which is used in the apparatus depicted in FIGURE 1;
FIGURE 3 is a perspective view of an absorbent product which has been fabricated by the apparatus depicted in FIGURES 1 and 2;
FIGURE 4 is a plan view of an insert and a flat screen which is used according to a second embodiment of the invention; and
FIGURE 5 is a plan view an absorbent product fabricated according to the invention which has an alternative contoured surface configuration.

### DETAILED DESCRIPTION OF THE PREFERRED EMBODIMENT(S)

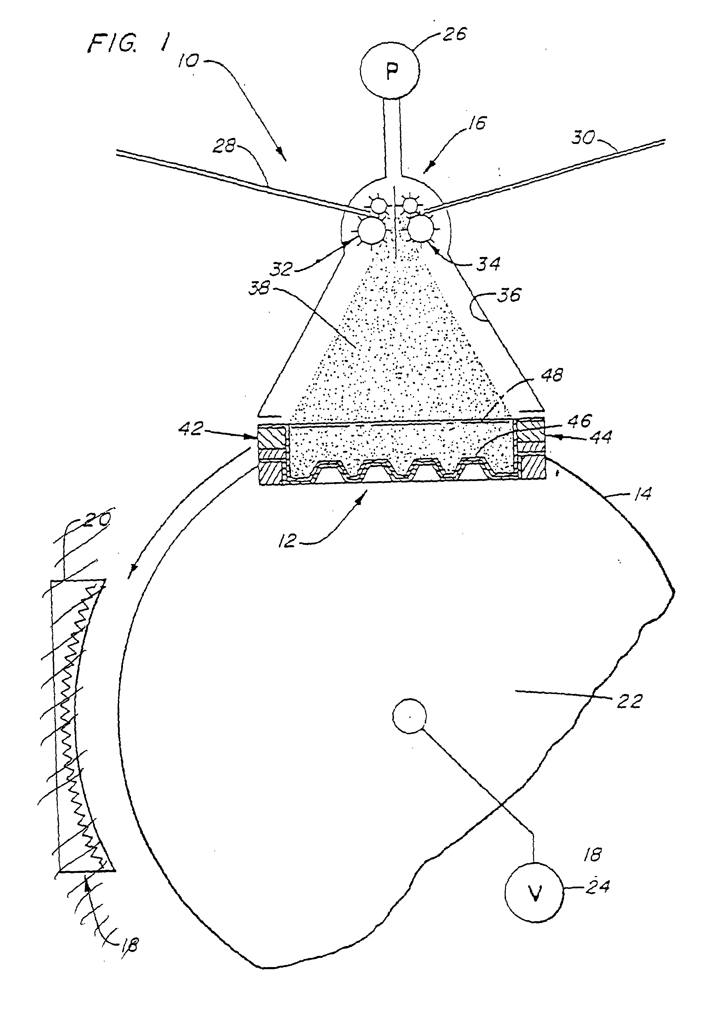

Referring now to the drawings, wherein like reference numerals designate corresponding structure throughout the views, and referring in particular to FIGURE 1, an apparatus 10 for manufacturing a three-dimensional fabric of the type which may be used in absorbent garments, dressings or the like includes a shaping assembly 12 which is mounted on a rotary conveyer 14. As is further shown in FIGURE 1, rotary conveyer 14 includes an internal chamber 22 which is in communication with a source 24 of vacuum. Fill station 16 includes a hood-like structure 36 defining a pressurized chamber 38 which is connected to a pressure source 26, shown at the top of FIGURE 1.

Referring again to FIGURE 1, a source of fiber 28 is fed into a first cutter 32 contained within hood-like structure 36. Similarly, a source of pulp 30 is fed into a second cutter 34, also contained within hood-like structure 36. Cutters 32. 4 are designed to cut fiber 28 and pulp 30, respectively into small pieces, which are mixed within hood-like structure 36 by their momentum and by airflow from pressure source 26 to settle within shaping assembly 12, as will be described in greater detail below.

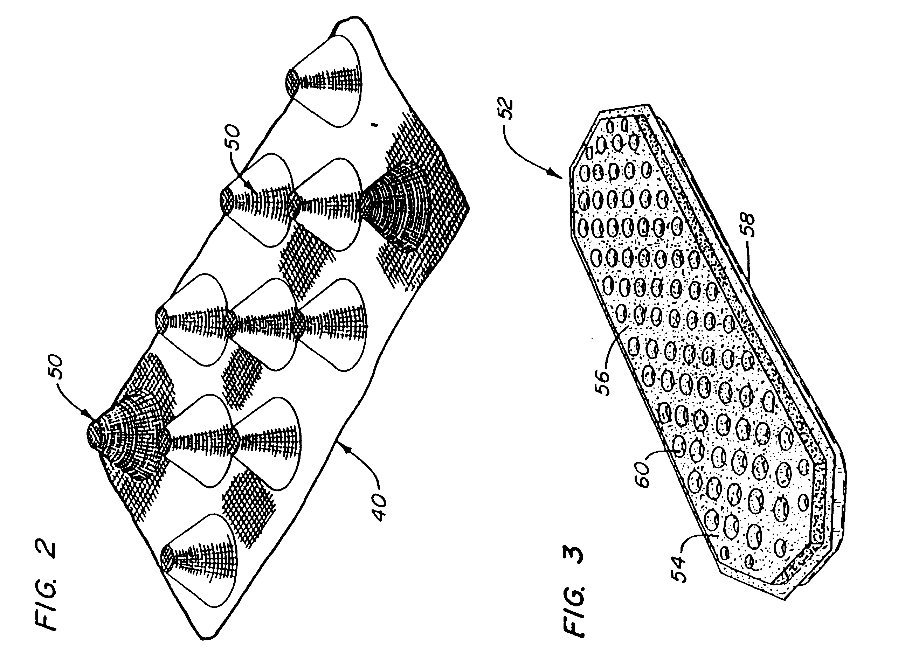

Looking now to FIGURES 1 and 2, shaping assembly 12 includes a contoured screen 40 which is mounted between a pair of oppositely facing mounting blocks 42, 44. In the preferred embodiment of the invention, as may be seen in FIGURE 2, contoured screen 40 is formed so as to have a pattern of spaced projections 50 formed thereon which are shaped as truncated cones. It is to be understood, however, that the exact shape of contoured screen 40 will vary according to the specific contour that is desired for the three-dimensional fabric being manufactured.

According the preferred embodiment of the invention, a cover 46 of air permeable material is positioned over the surface of contoured screen 40 which faces toward pressure source 26. The pressure differential between pressurized chamber 38 and internal chamber 22 induced by pressure source 26 and vacuum source 24 will cause cover 46 to conform to the surface of contoured screen 40, as shown in FIGURE 1.

During operation of the apparatus 10, the fragments of fiber 28 and pulp 30 will settle on top of the cover 46 which has assumed the shape of contoured screen 40, until a fill level 48 is reached. At that point, heat will melt a thermoplastic component of the fiber 28, thereby binding the fibers 28 to the fibers from pulp 30, and to the cover 46. Shaping assembly 12 is then permitted to cool, so that fibers 28, pulp 30 and cover 46 will solidify into a completed absorbent three-dimensional fabric.

The solidification process is be performed by blowin heated air through shaping assembly 12 at fill station 16 rather than at a separate heating station.

Pulp 30 may be substantially any type of absorbent pulp, such as wood fiber pulp. Most preferably, pulp 30 is Supersoft brand ELM pulp fiber, which is commercially obtainable from the International Paper Co. Fiber 28 can be any basic fiber that has a surface melt point (as in the case of a bico fiber) or melt point range (as in the case of a staple fiber) of 110°C to 150°C. The fibers 28 can be a bico, staple or adhesive fiber. A bico fiber would have a core with a higher melt point than its sheath, or two coextruded portions having different melting temperatures. Staple and adhesive fibers would have a melting point less than the temperature at which pulp 30 would begin to degrade or "yellow." Examples of commercially obtainable materials which could be used as the fiber 28 include Celbond brand #K54 polyester/copolyester fiber which is available from Hoechst Celanese Corporation, Celbond brand #255 fiber also available from Hoechst Celanese Corporation, DuPont binder fiber #D262 polyester/copolyester fiber, DuPont binder fiber #D270 polyester/copolyester fiber and adhesive fiber #410, which is available from the Eastman Chemical Company.

Cover 46 could be any type of material through which air can be forced, such as a woven cloth, a non-woven fabric, an unbonded non-woven fabric, or a perforated plastic material. Most preferably, cover 46 is fabricated from a fusible fiber non-woven fabric such as made from ENKA brand #1050, #1070 or #1045 fusible fiber, which are available from Johnson and Johnson Worldwide Absorbent Products, or a blend of Hollofil pulp and ENKA fibers which is available under the brand TDS from Johnson and Johnson Worldwide Absorbent Products.

It is to be understood that a three-dimensional fabric according to the invention could be manufactured by using cover 46 without fiber 28 or pulp 30. Alternatively, a three-dimensional fabric could be manufactured according to the invention by using fiber 28 without pulp 30 or cover 46, or with fiber 28 and cover 46 without the use of pulp 30.

In order to enhance the ability of cover 46 to conform to the textured surface of contoured screen 40; cover 46 may be crimped prior to its placement adjacent to contoured screen 40.

FIGURE 3 depicts an absorbent product 52, in this case a sanitary napkin, having a contoured absorbent surface 56 which has been fabricated according to the invention. As may be seen in FIGURE 3, contoured surface 56 includes a fabric cover 54 in which a plurality of depressions 60 have been formed. Depressions 60 conform to the projections 50 in the contoured screen 40 which is illustrated in FIGURE 2. A moisture barrier 58 is secured to cover 54 with a suitable adhesive to complete the fabrication of absorbent product 52.

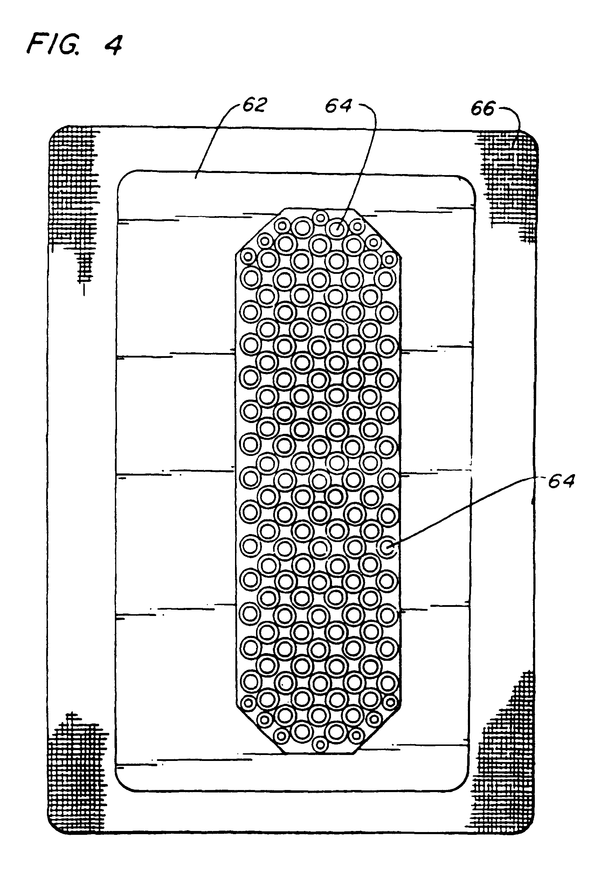

According to a second embodiment of the invention, depicted in FIGURE 4, a molded insert 62 having a contoured surface, in this case including holes 64, may be used in conjunction with a flat screen 66 in lieu of the contoured screen 40 in the embodiment depicted in FIGURES 1 and 2. Insert 62 may in some cases be more economical to manufacture than the contoured screen 40, although contoured screen 40 permits better air flow through the shaping assembly 12 during manufacturing of the three-dimensional fabric according to the invention.

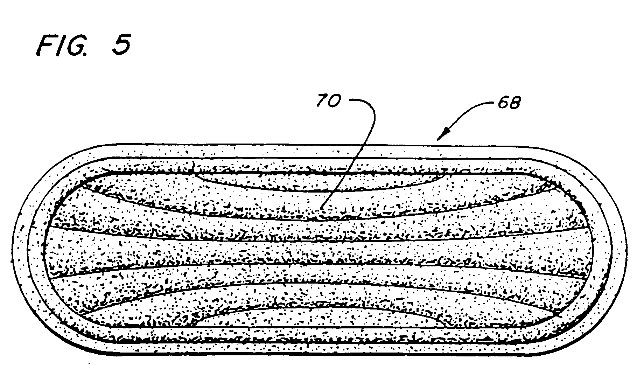

As shown in FIGURE 5, an absorbent product 68 can, by varying the shape of the contoured screen 40 or molded insert 62, be formed in substantially any desired shape. In this case, absorbent product 68 has a contoured surface 70 having a pattern of substantially longitudinal ribs and fluid-transporting valleys. For example, different contours may improve fluid transport, reduce fluid run off, minimize the contact surface of the fabric to a wound or vaginal wall to reduce removal forces, or be shaped so as to better conform to the anatomy of the consumer or patient.

It is to be understood, however, that even though numerous characteristics and advantages of the present invention have been set forth in the foregoing description, together with details of the structure and function of the invention, the disclosure is illustrative only, and changes may be made in detail, especially in matters of shape, size and arrangement of parts within the principles of the invention to the full extent indicated by the broad general meaning of the terms in which the appended claims are expressed.

## Claims

1. A method of manufacturing a three-dimensional fabric of the type which may be used in absorbent garments, dressings or the like, comprising the steps of:
(a) providing an apertured collector element having a predetermined three-dimensional shape;
(b) positioning a fabric material containing a thermobondable component adjacent to one side of said apertured collector element;
(c) developing a pressure differential between said one side of said apertured collector element and a second opposite side to force said fabric material against one side of said apertured collector element so that said fabric material conforms to said predetermined three-dimensional shape; and
(d) while maintaining said pressure differential, heating said so-conformed fabric material to melt the thermobondable component and then cooling to solidify the fabric material into said three-dimensional shape; charaterized by pressing a fabric material against said apertured collector element by heated air to position and solidify said fabric material into the desired shape in one step.

2. A method according to claim 1, wherein step (a) comprises providing a screen having said predetermined shape.

3. A method according to claim 1, wherein step (a) comprises providing a perforated insert having said predetermined shape.

4. A method according to claim 1, wherein step (b) comprises positioning a web of fabric adjacent to one side of said apertured collector element.

5. A method according to claim 4, wherein step (b) comprises positioning a web of non-woven fabric adjacent to one side of said apertured collector element.

6. A method according to claim 4, wherein step (b) comprises positioning a web of unbonded non-woven fabric adjacent to one side of said apertured collector element.

7. A method according to claim 4, wherein step (b) comprises positioning an apertured film adjacent to said one side of said apertured collector element.

8. A method according to claim 1, wherein step (b) further comprises collecting loose fabric fibers against said apertured collector element.

9. A method according to claim 1, wherein step (d) is performed by heating the fabric material.

10. A method according to claim 1, wherein step (d) is performed by forcing heated air through the fabric material.

11. An apparatus for manufacturing a three-dimensional fabric material which may be used in absorbent garments or dressings, comprising:
an apertured collector element having a predetermined three-dimensional shape;
means for positioning fabric material which contains a thermobondable component adjacent to one side of said apertured collector element;
means for developing a pressure differential between said one side of said apertured collector element and a second, opposite side to force said fabric material against said one side of said apertured collector element so that said fabric material conforms to said predetermined three-dimensional shape; arid
means for heating said so-conformed fabric material to melt the thermobondable component and then cooling to solidify the fabric material into said three-dimensional shape while said pressure differential is maintained,
wherein the means for developing a pressure differential and the means for heating are positioned to press said fabric material against said collector element and to solidify said fabric material with the use of heated gas.

12. An apparatus according to claim 11, wherein said apertured collector element comprises a screen which is contoured into said three-dimensional shape.

13. An apparatus according to claim 11, wherein said apertured collector element comprises a molded insert.

14. An apparatus according to claim 11, wherein said solidifying means comprises a heater.

## Patentansprüche

1. Verfahren zur Herstellung eines dreidimensionalen Vlieses von einem Typ, der für absorbierende Kleidungsstücke, Verbandstoffe oder dergleichen verwendet werden kann, mit den Schritten:
(a) Bereitstellen eines mit Löchern versehenen Sammelelementes in einer vorgegebenen dreidimensionalen Form;
(b) Anbringen eines Vliesmaterials, das wärmeverbindbare Bestandteile enthält, angrenzend an eine Seite des mit Löchern versehenen Sammelelementes;
(c) Erzeugen einer Druckdifferenz zwischen einer Seite des mit Löchern versehenen Sammelelementes und einer zweiten, entgegengesetzten Seite, um das Vliesmaterial gegen die eine Seite des mit Löchern versehenen Sammelelementes zu drükken, so daß das Vliesmaterial die vorgegebene dreidimensionale Form annimmt; und
(d) Aufheizen des auf diese Weise geformten Vliesmaterials unter Aufrechterhaltung der Druckdifferenz, um die wärmeverbindbaren Bestandteile zu schmelzen sowie anschließendes Abkühlen, um das Vliesmaterial in der dreidimensionalen Form zu verfestigen;
**gekennzeichnet durch**
Pressen eines Vliesmaterials gegen das mit Löchern versehene Sammelelement **durch** geheizte Luft, um das Vliesmaterial in die gewünschte Form in einem Schritt zu positionieren und zu verfestigen.

2. Verfahren nach Anspruch 1, bei welchem der Schritt (a) das Bereitstellen eines Siebes in der vorgegebenen Form umfaßt.

3. Verfahren nach Anspruch 1, bei welchem der Schritt (a) das Bereitstellen eines mit Löchern versehenen Einsatzes in der vorgegebenen Form umfaßt.

4. Verfahren nach Anspruch 1, bei welchem der Schritt (b) das Anbringen einer Bahn eines Vlieses angrenzend an die eine Seite des mit Löchern versehenen Sammelelementes umfaßt.

5. Verfahren nach Anspruch 4, bei welchem der Schritt (b) das Anbringen einer Bahn eines Faservlieses angrenzend an die eine Seite des mit Löchern versehenen Sammelelementes umfaßt.

6. Verfahren nach Anspruch 4, bei welchem der Schritt (b) das Anbringen einer Bahn eines nicht gebundenen Faservlieses angrenzend an die eine Seite des mit Löchern versehenen Sammelelementes umfaßt.

7. Verfahren nach Anspruch 4, bei welchem der Schritt (b) das Anbringen einer Bahn einer mit Löchern versehenen Folie angrenzend an die eine Seite des mit Löchern versehenen Sammelelementes umfaßt.

8. Verfahren nach Anspruch 1, bei welchem der Schritt (b) weiterhin das Sammeln loser Vliesfasern an dem mit Löchern versehenen Sammelelement umfaßt.

9. Verfahren nach Anspruch 1, bei welchem der Schritt (d) durch Aufheizen des Vliesmaterials durchgeführt wird.

10. Verfahren nach Anspruch 1, bei welchem der Schritt (d) durchgeführt wird, indem aufgeheizte Luft durch das Vliesmaterial gepreßt wird.

11. Vorrichtung zur Herstellung eines dreidimensionalen Vlieses von einem Typ, der für absorbierende Kleidungsstücke oder Verbandstoffe verwendet werden kann, mit:
einem mit Löchern versehenen Sammelelement mit einer vorgegebenen Form;
einer Einrichtung zum Positionieren von Vliesmaterial, welches wärmeverbindbare Bestandteile enthält, angrenzend an eine Seite des mit Löchern versehenen Sammelelementes;
einer Einrichtung zum Erzeugen einer Druckdifferenz zwischen einer Seite des mit Löchern versehenen Sammelelementes und einer zweiten, entgegengesetzten Seite, um das Vliesmaterial gegen die eine Seite des mit Löchern versehenen Sammelelementes zu drücken, so daß das Vliesmaterial die vorgegebene dreidimensionale Form annimmt; und
einer Einrichtung zum Aufheizen des auf diese Weise geformten Vliesmaterials, um die wärmeverbindbaren Bestandteile zu schmelzen, sowie anschließendes Abkühlen, um das Vliesmaterial in der dreidimensionalen Form zu verfestigen, wobei die Druckdifferenz aufrechterhalten wird;
wobei die Einrichtung zum Entwickeln einer Druckdifferenz und die Einrichtung zum Aufheizen so angeordnet sind, daß sie das Vliesmaterial gegen das Sammelelement pressen und das Vliesmaterial unter Verwendung des aufgeheizten Gases verfestigen.

12. Vorrichtung nach Anspruch 11, bei der das mit Löchern versehene Sammelelement ein Sieb aufweist, das in die dreidimensionale Form konturiert ist.

13. Vorrichtung nach Anspruch 11, bei der das mit Löchern versehene Sammelelement einen geformten Einsatz aufweist.

14. Vorrichtung nach Anspruch 11, bei der die Einrichtung zum Verfestigen einen Heizer aufweist.

## Revendications

1. Procédé de fabrication d'un tissu tridimensionnel du type qui peut être utilisé dans des vêtements absorbants, pansements, ou analogue, comportant les étapes consistant à :
(a) fournir un élément collecteur ouvert ayant une forme tridimensionnelle prédéterminée,
(b) positionner un matériau de tissu contenant un composant pouvant être thermiquement lié adjacent à un premier côté dudit élément collecteur ouvert,
(c) développer une différence de pression entre ledit premier côté dudit élément collecteur ouvert et un second côté opposé pour pousser ledit matériau de tissu contre le premier côté dudit élément collecteur ouvert de sorte que ledit matériau de tissu s'adapte à ladite forme tridimensionnelle prédéterminée, et
(d) tout en maintenant ladite différence de pression, chauffer ledit matériau en tissu ainsi conformé pour faire fondre le composant pouvant être thermiquement lié et ensuite refroidir pour solidifier le matériau en tissu dans ladite forme tridimensionnelle,
**caractérisé en ce qu'**on appuie un matériau de tissu contre ledit élément collecteur ouvert par de l'air chauffé pour positionner et solidifier ledit matériau de tissu dans la forme voulue en une étape.

2. Procédé selon la revendication 1, dans lequel l'étape (a) consiste à fournir un écran ayant ladite forme prédéterminée.

3. Procédé selon la revendication 1, dans lequel l'étape (a) consiste à fournir un élément rapporté perforé ayant ladite forme prédéterminée.

4. Procédé selon la revendication 1, dans lequel l'étape (b) consiste à positionner une bande de tissu adjacente au premier côté dudit élément collecteur ouvert.

5. Procédé selon la revendication 4, dans lequel l'étape (b) consiste à positionner une bande de tissu non-tissé adjacente au premier côté dudit élément collecteur ouvert.

6. Procédé selon la revendication 4, dans lequel l'étape (b) comporte le positionnement d'une bande de tissu non-tissé non-lié adjacente au premier côté dudit élément collecteur ouvert.

7. Procédé selon la revendication 4, dans lequel l'étape (b) comporte le positionnement d'un film à ouvertures adjacent audit premier côté dudit élément collecteur ouvert.

8. Procédé selon la revendication 1, dans lequel l'étape (b) comporte de plus l'étape consistant à collecter des fibres de tissu lâches contre ledit élément collecteur ouvert.

9. Procédé selon 1a revendication 1, dans lequel l'étape (d) est réalisée en chauffant le matériau de tissu.

10. Procédé selon la revendication 1, dans lequel l'étape (d) est réalisée en poussant de l'air chauffé à travers le matériau de tissu.

11. Dispositif destiné à fabriquer un matériau de tissu tridimensionnel qui peut être utilisé dans des vêtements absorbants ou des pansements, comportant :
un élément collecteur ouvert ayant une forme tridimensionnelle prédéterminée,
des moyens pour positionner le matériau de tissu qui comporte un composant pouvant être thermiquement lié adjacent à un premier côté dudit élément collecteur ouvert,
des moyens pour développer une différence de pression entre ledit premier côté dudit élément collecteur ouvert et un second côté opposé pour pousser ledit matériau de tissu contre ledit premier côté dudit élément collecteur ouvert de sorte que ledit matériau de tissu s'adapte à ladite forme tridimensionnelle prédéterminée, et
des moyens pour chauffer ledit matériau de tissu ainsi adapté pour faire fondre le composant pouvant être thermiquement lié et ensuite refroidir pour solidifier le matériau de tissu dans ladite forme tridimensionnelle pendant que ladite différence de pression est maintenue.

12. Dispositif selon la revendication 11, dans lequel ledit élément collecteur ouvert comporte un écran qui est profilé dans ladite forme tridimensionnelle.

13. Dispositif selon 1a revendication 11, dans lequel ledit élément collecteur ouvert comporte un élément rapporté moulé.

14. Dispositif selon 1a revendication 11, dans lequel lesdits moyens de solidification comportent un dispositif de chauffage.
